# EUROPEAN PATENT APPLICATION

(11) **EP 4 289 571 A1**
(43) Date of publication of application: **13.12.2023**
(21) Application number: 21924895.2
(22) Date of filing: 28.12.2021
(51) Int. Cl.: B25J 9/22, A61B 34/35

(54) **SURGERY ASSISTANCE ROBOT, SURGERY ASSISTANCE SYSTEM, AND METHOD FOR CONTROLLING SURGERY ASSISTANCE ROBOT**

(30) Priority: 05.02.2021 JP 2021017565
(71) Applicant: KAWASAKI JUKOGYO KABUSHIKI KAISHA, Kobe-shi, Hyogo 650-8670 (JP); Medicaroid Corporation, Hyogo 650-0047 (JP)
(72) Inventor: YAMAMORI, Hirofumi, Kobe-shi, Hyogo 650-8670 (JP); ICHII, Tetsuo, Kobe-shi, Hyogo 650-8670 (JP); KODAMA, Kazuki, Kobe-shi, Hyogo 650-8670 (JP)
(74) Representative: Hoffmann Eitle
(86) International application number: PCT/JP2021/048914
(87) International publication number: WO 2022/168510

(57) **Abstract**

A surgical robot includes a manipulator arm having a tip end side to which a surgical instrument is attached, a controller configured or programmed to perform a control to operate the manipulator arm to which the surgical instrument is attached, and a teaching unit to teach a teaching point in a space in which the manipulator arm operates. The controller is configured or programmed to set a virtual contact prohibited space in a space around a contact prohibited object based on a plurality of the teaching points taught using the teaching unit.

## Description

### Technical Field

The present disclosure relates to a surgical robot, a robotic surgical system, and a control method for a surgical robot, and more particularly, it relates to a surgical robot, a robotic surgical system, and a control method for a surgical robot that each prohibit a surgical instrument and a manipulator arm from contacting a contact prohibited object.

### Background Art

Conventionally, a surgical robot that prohibits a surgical instrument and a manipulator arm from contacting a contact prohibited object is known. Such a surgical robot is disclosed in Japanese Patent Laid-Open No. 2018-158155, for example.

Japanese Patent Laid-Open No. 2018-158155 discloses a robot system (surgical robot) including a manipulator arm having a tip end side to which a surgical instrument is attached and a control device that performs a control to operate the manipulator arm. In the robot system disclosed in Japanese Patent Laid-Open No. 2018-158155, the control device determines a surface of an obstacle (contact prohibited object) and performs a control to operate a manipulator so as to increase a distance between the manipulator arm and the surface of the obstacle.
Thus, the surgical instrument and the manipulator arm are prohibited from contacting the obstacle.

### Prior Art

### Patent Document

Patent Document 1: Japanese Patent Laid-Open No. 2018-158155

### Summary of the Invention

### Problem to be Solved by the Invention

However, in Japanese Patent Laid-Open No. 2018-158155, the control device defines the surface of the obstacle as a plane extending through a remote center (pivot position). Therefore, for example, when surgery is performed on the patient's abdomen, the plane passing through the remote center set on the abdomen is set as the surface of the obstacle, and thus it is difficult to set the periphery of the patient's face as an obstacle.

The present disclosure is intended to solve the above problem. The present disclosure aims to provide a surgical robot, a robotic surgical system, and a control method for a surgical robot each capable of improving the degree of freedom in setting a contact prohibited range for prohibiting a surgical instrument and a manipulator arm from contacting a contact prohibited object and easily setting the contact prohibited range.

### Means for Solving the Problem

In order to attain the aforementioned object, a surgical robot according to a first aspect of the present disclosure includes a manipulator arm having a tip end side to which a surgical instrument is attached, a controller configured or programmed to perform a control to operate the manipulator arm to which the surgical instrument is attached, and a teaching unit to teach a teaching point in a space in which the manipulator arm operates. The controller is configured or programmed to set a virtual contact prohibited space in a space around a contact prohibited object based on a plurality of the teaching points taught using the teaching unit.

In the surgical robot according to the first aspect of the present disclosure, as described above, the controller is configured or programmed to set the virtual contact prohibited space in the space around the contact prohibited object based on the teaching points taught using the teaching unit. Accordingly, unlike a case in which a plane passing through a pivot position is set as a surface of an obstacle based on the pivot position that serves as a fulcrum for movement of the surgical instrument, the contact prohibited space can be set at an arbitrary position with respect to the pivot position. Thus, the degree of freedom in setting a contact prohibited range for prohibiting the surgical instrument and the manipulator arm from contacting the contact prohibited object can be improved. Furthermore, the virtual contact prohibited space is set in the space around the contact prohibited object based on the teaching points taught using the teaching unit such that contact prohibited spaces having suitable shapes and sizes can be easily set for contact prohibited objects having various shapes and sizes. Consequently, the degree of freedom in setting the contact prohibited range for prohibiting the surgical instrument and the manipulator arm from contacting the contact prohibited object can be improved, and the contact prohibited range can be easily set.

A robotic surgical system according to a second aspect of the present disclosure includes a patient-side apparatus including a manipulator arm having a tip end side to which a surgical instrument is attached, and a teaching unit to teach a teaching point in a space in which the manipulator arm operates, an operator-side apparatus to receive an operation for the surgical instrument, and a controller configured or programmed to perform a control to operate the manipulator arm to which the surgical instrument is attached. The controller is configured or programmed to set a virtual contact prohibited space in a space around a contact prohibited object based on a plurality of the teaching points taught using the teaching unit.

In the robotic surgical system according to the second aspect of the present disclosure, as described above, the controller is configured or programmed to set the virtual contact prohibited space in the space around the contact prohibited object based on the teaching points taught using the teaching unit. Accordingly, unlike a case in which a plane passing through a pivot position is set as a surface of an obstacle based on the pivot position that serves as a fulcrum for movement of the surgical instrument, the contact prohibited space can be set at an arbitrary position with respect to the pivot position. Thus, the degree of freedom in setting a contact prohibited range for prohibiting the surgical instrument and the manipulator arm from contacting the contact prohibited object can be improved. Furthermore, the virtual contact prohibited space is set in the space around the contact prohibited object based on the teaching points taught using the teaching unit such that contact prohibited spaces having suitable shapes and sizes can be easily set for contact prohibited objects having various shapes and sizes. Consequently, it is possible to provide the robotic surgical system capable of improving the degree of freedom in setting the contact prohibited range for prohibiting the surgical instrument and the manipulator arm from contacting the contact prohibited object, and easily setting the contact prohibited range.

A control method for a surgical robot according to a third aspect of the present disclosure includes setting a virtual contact prohibited space in a space around a contact prohibited object based on a plurality of teaching points taught using a teaching unit to teach the teaching points in a space in which a manipulator arm having a tip end side to which a surgical instrument is attached operates, and controlling operation of the manipulator arm such that the surgical instrument and the manipulator arm do not enter the set contact prohibited space.

As described above, the control method for a surgical robot according to the third aspect of the present disclosure includes setting the virtual contact prohibited space in the space around the contact prohibited object based on the teaching points taught using the teaching unit. Accordingly, unlike a case in which a plane passing through a pivot position is set as a surface of an obstacle based on the pivot position that serves as a fulcrum for movement of the surgical instrument, the contact prohibited space can be set at an arbitrary position with respect to the pivot position. Thus, the degree of freedom in setting a contact prohibited range for prohibiting the surgical instrument and the manipulator arm from contacting the contact prohibited object can be improved. Furthermore, the virtual contact prohibited space is set in the space around the contact prohibited object based on the teaching points taught using the teaching unit such that contact prohibited spaces having suitable shapes and sizes can be easily set for contact prohibited objects having various shapes and sizes. Consequently, it is possible to provide the control method for a surgical robot capable of improving the degree of freedom in setting the contact prohibited range for prohibiting the surgical instrument and the manipulator arm from contacting the contact prohibited object, and easily setting the contact prohibited range.

### Effect of the Invention

According to the present disclosure, as described above, it is possible to improve the degree of freedom in setting the contact prohibited range for prohibiting the surgical instrument and the manipulator arm from contacting the contact prohibited object, and easily set the contact prohibited range.

### Brief Description of the Drawings

FIG. 1 is a diagram showing the configuration of a robotic surgical system according to an embodiment.
FIG. 2 is a diagram showing the configuration of a medical manipulator according to the embodiment.
FIG. 3 is a diagram showing the configuration of a manipulator arm of the medical manipulator according to the embodiment.
FIG. 4 is a diagram showing a pair of forceps.
FIG. 5 is a perspective view showing the configuration of an operation unit of the medical manipulator according to the embodiment.
FIG. 6 is a diagram showing an endoscope.
FIG. 7 is a diagram showing a pivot position teaching instrument.
FIG. 8 is a diagram for illustrating a pivot position.
FIG. 9 is a block diagram showing the configuration of a controller of the medical manipulator according to the embodiment.
FIG. 10 is a diagram showing rotation axes and a linear motion axis of the manipulator arm.
FIG. 11 is a diagram showing rotation axes and the linear motion axis of a translation mechanism and a surgical instrument.
FIG. 12 is a diagram showing an example of a contact prohibited space according to the embodiment.
FIG. 13 is a diagram showing an example of a setting screen for the contact prohibited space according to the embodiment.
FIG. 14 is a flowchart for illustrating a procedure for setting the contact prohibited space according to the embodiment.
FIG. 15 is a flowchart for illustrating a procedure for confirming the contact prohibited space according to the embodiment.
FIG. 16 is a diagram for illustrating confirmation of the contact prohibited space according to the embodiment. Modes for Carrying Out the Invention

An embodiment of the present disclosure is hereinafter described on the basis of the drawings.

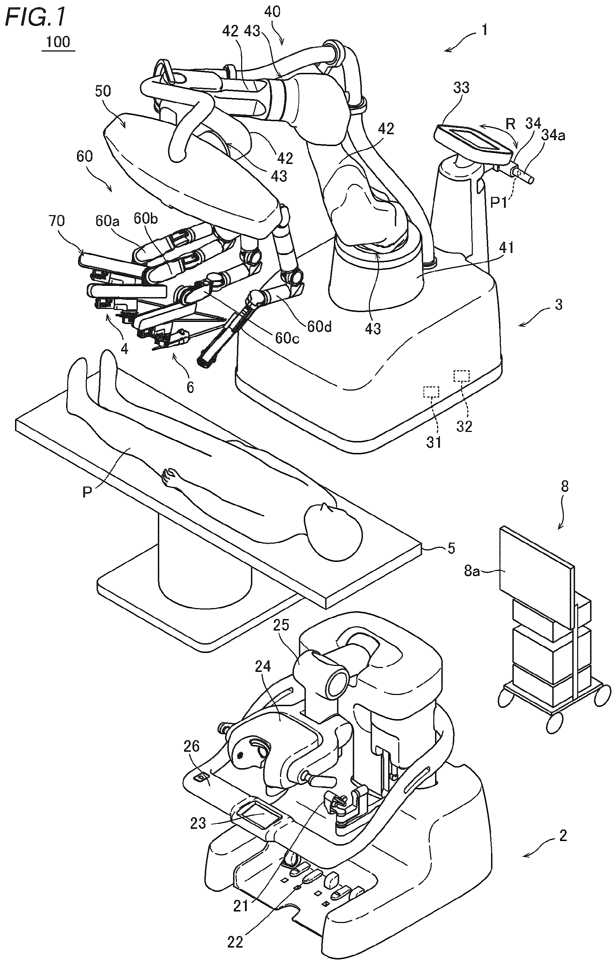

The configuration of a robotic surgical system 100 according to the embodiment is now described with reference to FIGS. 1 to 15. The robotic surgical system 100 includes a medical manipulator 1 that is a patient P-side apparatus and a remote control apparatus 2 that is an operator-side apparatus to operate the medical manipulator 1. The medical manipulator 1 includes a medical cart 3, and is movable. The remote control apparatus 2 is spaced apart from the medical manipulator 1, and the medical manipulator 1 is remotely operated by the remote control apparatus 2. A surgeon inputs a command to the remote control apparatus 2 to cause the medical manipulator 1 to perform a desired operation. The remote control apparatus 2 transmits the input command to the medical manipulator 1. The medical manipulator 1 operates based on the received command. The medical manipulator 1 is arranged in an operating room that is a sterilized sterile field. The medical manipulator 1 is an example of a "surgical robot" or "patient-side apparatus" in the claims. The remote control apparatus 2 is an example of an "operator-side apparatus" in the claims.

The remote control apparatus 2 is arranged inside or outside the operating room, for example. The remote control apparatus 2 includes operation manipulator arms 21, operation pedals 22, a touch panel 23, a monitor 24, a support arm 25, and a support bar 26. The operation manipulator arms 21 include operation handles for the surgeon to input commands. The operation manipulator arms 21 receive the amount of operation for a surgical instrument 4. The monitor 24 is a scope-type display that displays an image captured by an endoscope 6. The support arm 25 supports the monitor 24 so as to align the height of the monitor 24 with the height of the surgeon's face. The touch panel 23 is arranged on the support bar 26. The surgeon's head is detected by a sensor (not shown) provided in the vicinity of the monitor 24 such that the medical manipulator 1 can be operated by the remote control apparatus 2. The surgeon operates the operation manipulator arms 21 and the operation pedals 22 while visually recognizing an affected area on the monitor 24. Thus, a command is input to the remote control apparatus 2. The command input to the remote control apparatus 2 is transmitted to the medical manipulator 1. The touch panel 23 is an example of an "input" in the claims.

The medical cart 3 includes a controller 31 that controls the operation of the medical manipulator 1 and a storage 32 that stores programs or the like to control the operation of the medical manipulator 1. The controller 31 of the medical cart 3 controls the operation of the medical manipulator 1 based on the command input to the remote control apparatus 2.

The medical cart 3 includes an input device 33. The input device 33 receives operations to move a positioner 40, an arm base 50, and a plurality of manipulator arms 60 or change their postures mainly in order to prepare for surgery before the surgery. The input device 33 is an example of an "input" in the claims.

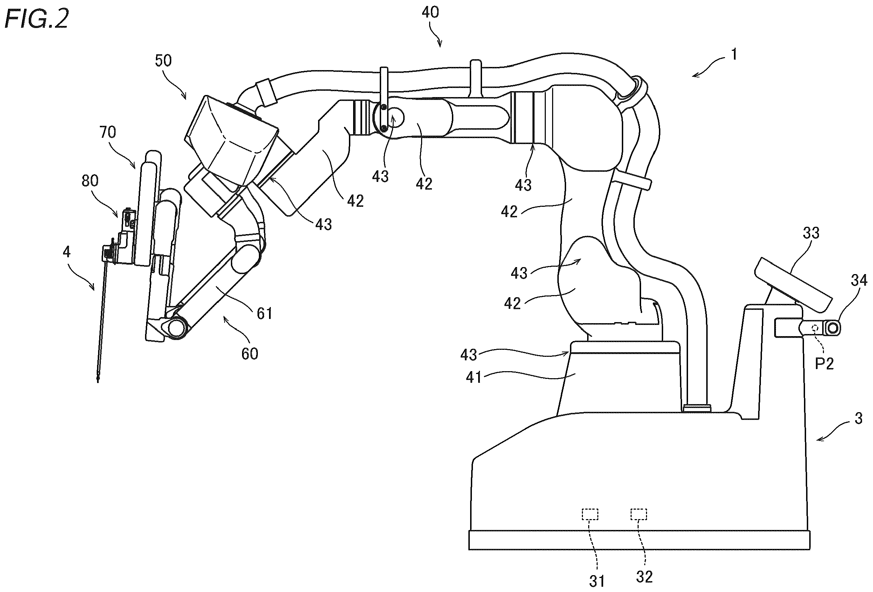

The medical manipulator 1 shown in FIGS. 1 and 2 is arranged in the operating room. As shown in FIG. 1, the medical manipulator 1 performs surgery on a patient P on an operating table 5. The medical manipulator 1 includes the medical cart 3, the positioner 40, the arm base 50, and the plurality of manipulator arms 60. The arm base 50 is attached to the tip end of the positioner 40. The arm base 50 has a relatively long rod shape (long shape). The base of each of the plurality of manipulator arms 60 is coupled to a slide belt (not shown) inside the arm base. The slide belt is stretched over a plurality of pulleys and a drive motor (not shown) provided in the arm base, and the slide belt is rotated such that the base of each of the manipulator arms 60 is moved. In other words, the arm base 50 can adjust a distance between the manipulator arms 60 based on a command from the controller 31. Each of the plurality of manipulator arms 60 is able to take a folded posture (stored posture). The arm base 50 and the plurality of manipulator arms 60 are covered with sterile drapes (not shown) and used.

The positioner 40 includes a 7-axis articulated robot, for example. The positioner 40 is arranged on the medical cart 3. The positioner 40 moves the arm base 50. Specifically, the positioner 40 moves the position of the arm base 50 three-dimensionally.

The positioner 40 includes a base 41 and a plurality of links 42 coupled to the base 41. The plurality of links 42 are coupled to each other by joints 43.

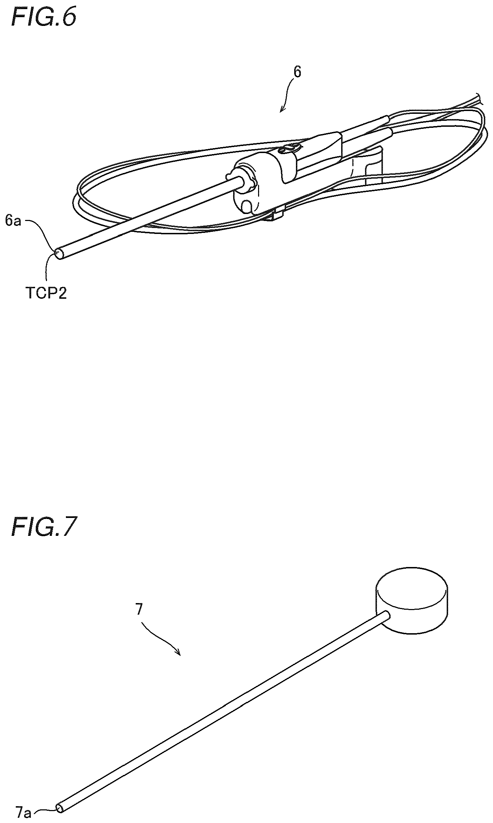

As shown in FIG. 1, the surgical instrument 4 is attached to the tip end of each of the plurality of manipulator arms 60. The surgical instrument 4 includes a replaceable instrument or the endoscope 6 (see FIG. 6), for example. The endoscope 6 is an example of a "teaching member" or a "surgical instrument" in the claims.

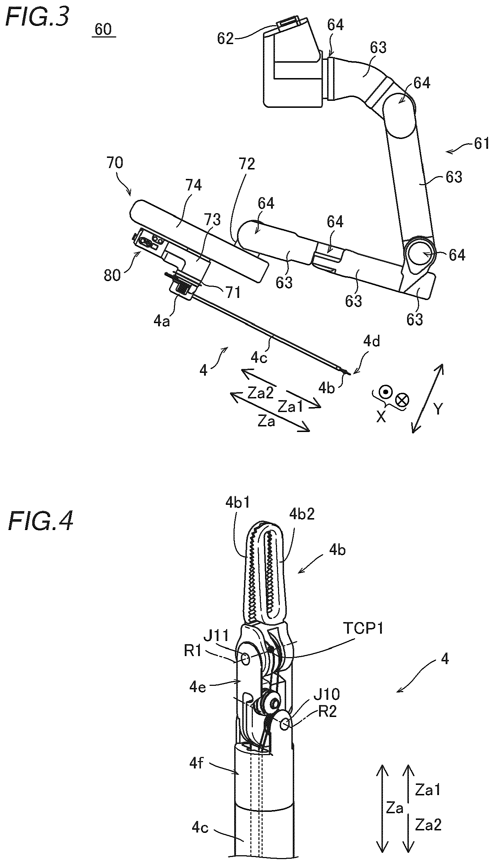

As shown in FIG. 3, the instrument includes a driven unit 4a driven by servomotors M2 provided in a holder 71 of each of the manipulator arms 60. A pair of forceps 4b is provided at the tip end of the instrument as an example of an end effector. The end effector includes a pair of forceps, a pair of scissors, a grasper, a needle holder, a microdissector, a stable applier, a tacker, a suction cleaning tool, a snare wire, a clip applier, etc. as instruments having joints. The end effector includes a cutting blade, a cautery probe, a washer, a catheter, a suction orifice, etc. as instruments having no joint. The pair of forceps 4b includes two end effector members 4b1 and 4b2.

As shown in FIG. 4, the instrument includes a first support 4e that supports the base end sides of the end effector members 4b1 and 4b2 such that the base end sides of the end effector members 4b1 and 4b2 are rotatable about a J11 axis, a second support 4f that supports the base end side of the first support 4e such that the base end side of the first support 4e is rotatable about a J10 axis, and a shaft 4c connected to the base end side of the second support 4f. The driven unit 4a, the shaft 4c, the second support 4f, the first support 4e, and the pair of forceps 4b are arranged along a Za direction. The J11 axis is orthogonal to a direction (Za direction) in which the shaft 4c extends. The J10 axis is spaced apart from the J11 axis in the direction in which the shaft 4c extends, and is orthogonal to the direction in which the shaft 4c extends and the J11 axis.

The pair of forceps 4b is attached to the first support 4e so as to rotate about the rotation axis R1 of the J11 axis. The second support 4f supports the first support 4e such that the first support 4e is rotatable about the J10 axis. That is, the first support 4e is attached to the second support 4f so as to rotate about the rotation axis R2 of the J10 axis. A portion of the first support 4e on the tip end side (Zal direction side) has a U-shape. A tool center point (TCP1, clevis) is set at the center of the tip end of the U-shaped portion of the first support 4e in a rotation axis R1 direction.

As shown in FIG. 6, a TCP2 of the endoscope 6 is set at the tip end of the endoscope 6. The endoscope 6 is attached to the tip end side of the manipulator arm 60 to image a surgical site. The endoscope 6 includes a stereo camera, for example, and can capture a 3D image of the surgical site.

The configuration of the manipulator arms 60 is now described in detail.

As shown in FIG. 3, each of the manipulator arms 60 includes an arm portion 61 (a base 62, links 63, and joints 64) and a translation mechanism 70 provided at the tip end of the arm portion 61. The tip end sides of the manipulator arms 60 three-dimensionally move with respect to the base sides (arm base 50) of the manipulator arms 60. The plurality of manipulator arms 60 have the same or similar configuration as each other.

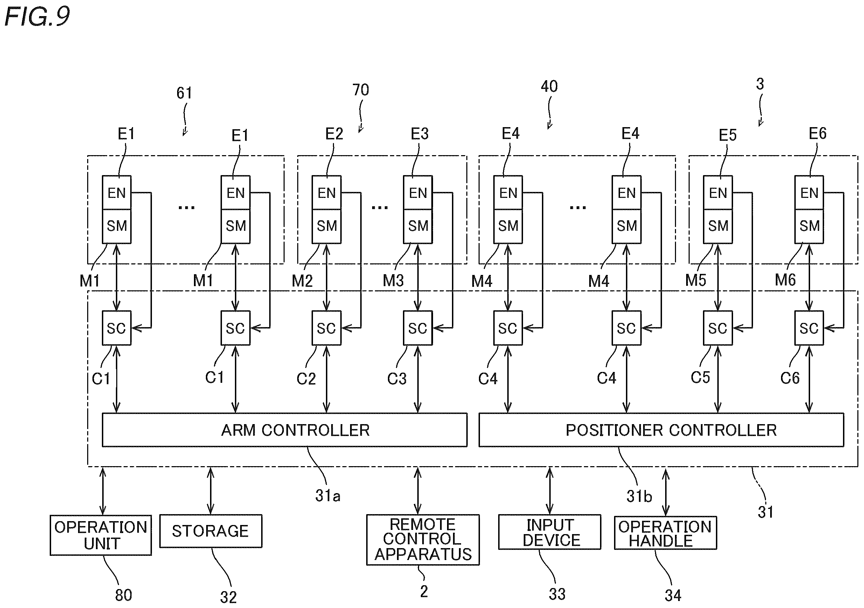

The translation mechanism 70 is provided on the tip end side of the arm portion 61, and the surgical instrument 4 is attached thereto. The translation mechanism 70 translates the surgical instrument 4 in a direction in which the surgical instrument 4 is inserted into the patient P. Furthermore, the translation mechanism 70 translates the surgical instrument 4 relative to the arm portion 61. Specifically, the translation mechanism 70 includes the holder 71 that holds the surgical instrument 4. The servomotors M2 (see FIG. 9) are housed in the holder 71. The servomotors M2 rotate rotary bodies provided in the driven unit 4a of the surgical instrument 4. The rotary bodies in the driven unit 4a are rotated such that the pair of forceps 4b is operated.

The arm portion 61 includes a 7-axis articulated robot arm. The arm portion 61 includes the base 62 to attach the arm portion 61 to the arm base 50, and a plurality of links 63 coupled to the base 62. The plurality of links 63 are coupled to each other by the joints 64.

The translation mechanism 70 translates the surgical instrument 4 attached to the holder 71 along the Za direction (the direction in which the shaft 4c extends) by translating the holder 71 along the Za direction. Specifically, the translation mechanism 70 includes a base end side link 72 connected to the tip end of the arm portion 61, a tip end side link 73, and a coupling link 74 provided between the base end side link 72 and the tip end side link 73. The holder 71 is provided on the tip end side link 73.

The coupling link 74 of the translation mechanism 70 is configured as a double speed mechanism that moves the tip end side link 73 relative to the base end side link 72 along the Za direction. The tip end side link 73 is moved along the Za direction relative to the base end side link 72 such that the surgical instrument 4 provided on the holder 71 is translated along the Za direction. The tip end of the arm portion 61 is connected to the base end side link 72 so as to rotate the base end side link 72 about an X direction orthogonal to the Za direction.

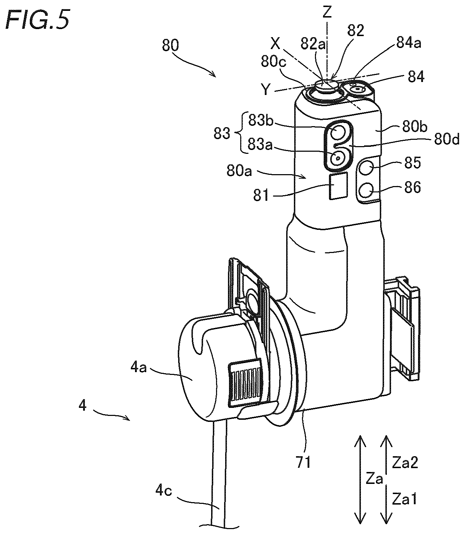

As shown in FIG. 5, the medical manipulator 1 includes an operation unit 80 provided on each of the manipulator arms 60 to operate the manipulator arm 60. The operation unit 80 includes enable switches 81, a joystick 82, and switch units 83. The enable switches 81 enable or disable movement of the manipulator arm 60 in response to the joystick 82 and the switch units 83. The enable switches 81 enable movement of the surgical instrument 4 by the manipulator arm 60 when the enable switches 81 are pressed by an operator (such as a nurse or an assistant) grasping the operation unit 80. A pair of enable switches 81 are provided on opposite sides of the outer peripheral surface 80a of the operation unit 80. The joystick 82 can manipulate the moving direction of the manipulator arm 60. The operation unit 80 is an example of a "teaching unit" in the claims.

Each of the switch units 83 includes a switch 83a to move the surgical instrument 4 in the direction in which the surgical instrument 4 is inserted into the patient P along the longitudinal direction of the surgical instrument 4, and a switch 83b to move the surgical instrument 4 in a direction opposite to the direction in which the surgical instrument 4 is inserted into the patient P. Both the switch 83a and the switch 83b are push-button switches. The switch units 83 are provided on the opposite sides of the outer peripheral surface 80a of the operation unit 80. Specifically, each (a pair of switches 83a and 83b) of the switch units 83 is provided on each of opposite side surfaces of the operation unit 80.

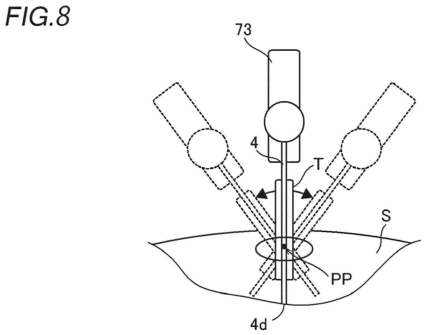

As shown in FIG. 5, the operation unit 80 includes pivot buttons 85 to teach a pivot position PP that serves as a fulcrum (see FIG. 8) for movement of the surgical instrument 4 attached to the manipulator arm 60. The pivot buttons 85 are provided adjacent to the enable switches 81 on surfaces 80b of the operation unit 80. The pivot buttons 85 are pressed while the tip end 6a of the endoscope 6 (see FIG. 6) or the tip end 7a of a pivot teaching member 7 (see FIG. 7) is moved to a position corresponding to the insertion position of a trocar T inserted into the body surface S of the patient P such that the pivot position PP is taught and stored in the storage 32. In the teaching of the pivot position PP, the pivot position PP is set as one point (coordinates), and the direction of the surgical instrument 4 is not set. A pair of pivot buttons 85 are provided on the opposite sides of the outer peripheral surface 80a of the operation unit 80. The pivot teaching member 7 is an example of a "teaching unit" in the claims.

As shown in FIG. 7, the pivot teaching member 7 is attached to the tip end side of the manipulator arm 60 and teaches the pivot position PP (see FIG. 8) that serves as a fulcrum for movement of the surgical instrument 4.

As shown in FIG. 1, the endoscope 6 is attached to one manipulator arm 60 (manipulator arm 60c, for example) of the plurality of manipulator arms 60, and surgical instruments 4 other than the endoscope 6 are attached to the remaining manipulator arms 60 (manipulator arms 60a, 60b, and 60d, for example). Specifically, in surgery, the endoscope 6 is attached to one of four manipulator arms 60, and the surgical instruments 4 (such as pairs of forceps) other than the endoscope 6 are attached to the three manipulator arms 60. A pivot position PP1 is taught with the endoscope 6 attached to the manipulator arm 60 to which the endoscope 6 is to be attached. Furthermore, a pivot position PP2 is taught with the pivot teaching member 7 attached to the manipulator arm 60 to which the surgical instrument 4 other than the endoscope 6 is to be attached. The endoscope 6 is attached to one of two manipulator arms 60 (manipulator arms 60b and 60c) arranged in the center among the four manipulator arms 60 arranged adjacent to each other. That is, the pivot position PP is individually set for each of the plurality of manipulator arms 60.

As shown in FIG. 5, adjustment buttons 86 are provided on the surfaces 80b of the operation unit 80 to optimize the position of the manipulator arm 60. After the pivot position PP for the manipulator arm 60 to which the endoscope 6 has been attached is taught, the adjustment buttons 86 are pressed such that the positions of the other manipulator arms 60 (arm base 50) are optimized. A pair of adjustment buttons 86 are provided on the opposite sides of the outer peripheral surface 80a of the operation unit 80.

As shown in FIG. 5, the operation unit 80 includes a mode switching button 84 to switch between a mode for translating the surgical instrument 4 (or the endoscope 6) attached to the arm manipulator 60 and a mode for rotationally moving the surgical instrument 4 (or the endoscope 6). Furthermore, a mode indicator 84a is provided in the vicinity of the mode switching button 84. The mode indicator 84a indicates a switched mode. Specifically, the mode indicator 84a is turned on (rotational movement mode) or off (translational mode) such that the current mode (translational mode or rotational movement mode) is indicated.

The mode indicator 84a also serves as a pivot position indicator that indicates that the pivot position PP has been taught.

In the mode for translating the manipulator arm 60, the manipulator arm 60 is moved such that the tip end 4d of the surgical instrument 4 moves on an X-Y plane. In the mode for rotationally moving the manipulator arm 60, when the pivot position PP is not taught, the manipulator arm 60 is moved such that the surgical instrument 4 rotationally moves about the pair of forceps 4b, and when the pivot position PP is taught, the manipulator arm 60 is moved such that the surgical instrument 4 rotationally moves about the pivot position PP as a fulcrum. The surgical instrument 4 is rotationally moved while the shaft 4c of the surgical instrument 4 is inserted into the trocar T.

As shown in FIG. 9, the manipulator arm 60 includes a plurality of servomotors M1, encoders E1, and speed reducers (not shown) so as to correspond to a plurality of joints 64 of the arm portion 61. The encoders E1 detect the rotation angles of the servomotors M1. The speed reducers slow down rotation of the servomotors M1 to increase the torques.

As shown in FIG. 9, the translation mechanism 70 includes the servomotors M2 to rotate the rotary bodies provided in the driven unit 4a of the surgical instrument 4, a servomotor M3 to translate the surgical instrument 4, encoders E2, an encoder E3, and speed reducers (not shown). The encoders E2 and E3 detect the rotation angles of the servomotors M2 and M3, respectively. The speed reducers slow down rotation of the servomotors M2 and M3 to increase the torques.

The positioner 40 includes a plurality of servomotors M4, encoders E4, and speed reducers (not shown) so as to correspond to a plurality of joints 43 of the positioner 40. The encoders E4 detect the rotation angles of the servomotors M4. The speed reducers slow down rotation of the servomotors M4 to increase the torques.

The medical cart 3 includes servomotors M5 to drive a plurality of front wheels (not shown) of the medical cart 3, respectively, encoders E5, speed reducers (not shown), and brakes (not shown). The encoders E5 detect the rotation angles of the servomotors M5. The speed reducers slow down rotation of the servomotors M5 to increase the torques.

A potentiometer P1 (see FIG. 1) is provided on a throttle 34a of the medical cart 3, and the servomotors M5 of the front wheels are driven based on a rotation angle detected by the potentiometer P1 according to the twist of the throttle 34a. Rear wheels (not shown) of the medical cart 3 are of the dual wheel type, and the rear wheels are steered based on rightward-leftward (R direction) rotation of an operation handle 34. Furthermore, a potentiometer P2 (see FIG. 2) is provided on the operation handle 34 of the medical cart 3, and servomotors M6, encoders E6, and speed reducers (not shown) are provided on the rear wheels of the medical cart 3. The speed reducers slow down rotation of the servomotors M6 to increase the torques. The servomotors M6 are driven based on a rotation angle detected by the potentiometer P2 according to rightward-leftward (R direction) rotation of the operation handle 34. That is, steering of the rear wheels by the rightward-leftward (R direction) rotation of the operation handle 34 is power-assisted by the servomotors M6.

The front wheels of the medical cart 3 are driven such that the medical cart 3 moves in a forward-rearward direction. Furthermore, the operation handle 34 of the medical cart 3 is rotated such that the rear wheels are steered, and the medical cart 3 turns in a rightward-leftward direction.

The controller 31 of the medical cart 3 includes an arm controller 31a to control movement of the plurality of manipulator arms 60 based on commands, and a positioner controller 31b to control movement of the positioner 40 and driving of the front wheels and rear wheels (not shown) of the medical cart 3 based on commands. Servo controllers C1 that control the servomotors M1 to drive the manipulator arm 60 are electrically connected to the arm controller 31a. The encoders E1 that detect the rotation angles of the servomotors M1 are electrically connected to the servo controllers C1.

Servo controllers C2 that control the servomotors M2 to drive the surgical instrument 4 are electrically connected to the arm controller 31a. The encoders E2 that detect the rotation angles of the servomotors M2 are electrically connected to the servo controllers C2. A servo controller C3 that controls the servomotor M3 to translate the translation mechanism 70 is electrically connected to the arm controller 31a. The encoder E3 that detects the rotation angle of the servomotor M3 is electrically connected to the servo controller C3.

An operation command input to the remote control apparatus 2 is input to the arm controller 31a. The arm controller 31a generates position commands based on the input operation command and the rotation angles detected by the encoders E1 (E2, E3), and outputs the position commands to the servo controllers C1 (C2, C3). The servo controllers C1 (C2, C3) generate torque commands based on the position commands input from the arm controller 31a and the rotation angles detected by the encoders E1 (E2, E3), and output the torque commands to the servomotors M1 (M2, M3). Thus, the manipulator arm 60 is moved according to the operation command input to the remote control apparatus 2.

The arm controller 31a operates the manipulator arm 60 based on an input signal from the joystick 82 of the operation unit 80. Specifically, the arm controller 31a generates position commands based on the input signal (operation command) input from the joystick 82 and the rotation angles detected by the encoders E1, and outputs the position commands to the servo controllers C1. The servo controllers C1 generate torque commands based on the position commands input from the arm controller 31a and the rotation angles detected by the encoders E1, and output the torque commands to the servomotors M1. Thus, the manipulator arm 60 is moved according to the operation command input to the joystick 82.

The arm controller 31a operates the manipulator arm 60 based on an input signal from each of the switch units 83 of the operation unit 80. Specifically, the arm controller 31a generates a position command based on the input signal (operation command) input from each of the switch units 83 and the rotation angle detected by the encoders E1 or the encoder E3, and outputs the position command to the servo controllers C1 or the servo controller C3. The servo controllers C1 or the servo controller C3 generates a torque command based on the position command input from the arm controller 31a and the rotation angle detected by the encoders E1 or the encoder E3, and outputs the torque command to the servomotors M1 or the servomotor M3. Thus, the manipulator arm 60 is moved according to the operation command input to each of the switch units 83.

As shown in FIG. 9, servo controllers C4 that control the servomotors M4 to move the positioner 40 are electrically connected to the positioner controller 31b. The encoders E4 that detect the rotation angles of the servomotors M4 are electrically connected to the servo controllers C4. Servo controllers C5 that control the servomotors M5 to drive the front wheels (not shown) of the medical cart 3 are electrically connected to the positioner controller 31b. The encoders E5 that detect the rotation angles of the servomotors M5 are electrically connected to the servo controllers C5. Servo controllers C6 that control the servomotors M6 to drive the rear wheels (not shown) of the medical cart 3 are electrically connected to the positioner controller 31b. The encoders E6 that detect the rotation angles of the servomotors M6 are electrically connected to the servo controllers C6.

An operation command is input from the input device 33 to the positioner controller 31b. The positioner controller 31b generates position commands based on the operation command input from the input device 33 and the rotation angles detected by the encoders E4, and outputs the position commands to the servo controllers C4. The servo controllers C4 generate torque commands based on the position commands input from the positioner controller 31b and the rotation angles detected by the encoders E4, and output the torque commands to the servomotors M4. Thus, the positioner 40 is moved according to the operation command input to the input device 33. Although detailed description is omitted, the positioner controller 31b moves the medical cart 3 based on an operation command from the operation handle 34 by a similar procedure.

The robotic surgical system 100 includes a monitor cart 8, as shown in FIG. 1. The monitor cart 8 includes a display 8a. The same image as that displayed on the monitor 24 of the remote control apparatus 2 is displayed on the display 8a of the monitor cart 8. That is, the image displayed on the monitor 24 and viewed by the surgeon can be viewed by the operator (such as a nurse or an assistant) around the medical manipulator 1 and the patient P on the display 8a of the monitor cart 8.

### Axes of Manipulator Arm

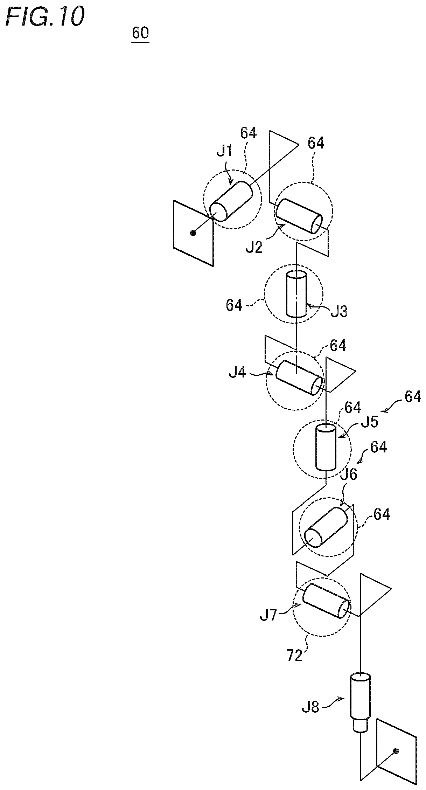

Axes of the manipulator arm 60 are now described with reference to FIG. 10.

In this embodiment, as shown in FIG. 10, the manipulator arm 60 includes seven or more joint axes (eight axes in this embodiment). Specifically, the manipulator arm 60 includes J1 to J7 axes as rotation axes and a J8 axis as a linear motion axis. The J1 to J7 axes correspond to the rotation axes of the joints 64 of the arm portion 61. The J7 axis also corresponds to the base end side link 72 of the translation mechanism 70 (see FIG. 3). The J8 axis corresponds to an axis for moving the tip end side link 73 of the translation mechanism 70 relative to the base end side link 72 along the Za direction (see FIG. 3). That is, the servomotors M1 shown in FIG. 9 are provided so as to correspond to the J1 to J7 axes of the manipulator arm 60. The servomotor M3 is provided so as to correspond to the J8 axis.

### Axes of Surgical Instrument (Pair of Forceps)

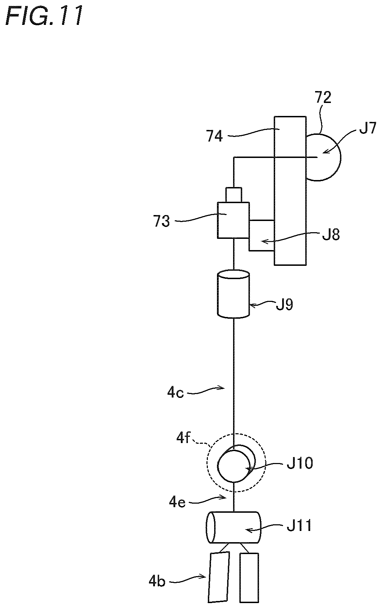

Axes of the surgical instrument 4 (pair of forceps 4b) are now described with reference to FIG. 11.

As shown in FIG. 11, the surgical instrument 4 (pair of forceps 4b) includes a J9 axis as a rotation axis (an axis along the direction in which the shaft 4c extends) of the shaft 4c, the J10 axis as a rotation axis of the second support 4f connected to the shaft 4c, the J11 axis as an axis about which the pair of forceps 4b rotates with respect to the first support 4e, and a J12 axis as an opening/closing axis of the pair of forceps 4b. A plurality of (four, for example) servomotors M2 provided in the holder 71 of the manipulator arm 60 are provided, and the driven unit 4a is driven by the plurality of servomotors M2. Thus, the surgical instrument 4 is driven around the J9 to J12 axes.

### Configuration of Controller to Perform Contact Prohibition Control for Manipular Arm

The specific configuration of the controller 31 for a contact prohibition control to operate the manipulator arm 60 such that the surgical instrument 4 and the manipulator arm 60 do not enter a contact prohibited space VPC is now described. The controller 31 performs a control to operate the surgical instrument 4 based on an operation received by the operation manipulator arm 21.

The controller 31 performs a control to operate the manipulator arm 60 to which the surgical instrument 4 is attached. Furthermore, the controller 31 sets a virtual contact prohibited space VPC in a space around a contact prohibited object (patient P) based on a teaching point TP taught using the endoscope 6 or the pivot teaching member 7 and the operation unit 80. The controller 31 also performs the contact prohibition control to operate the manipulator arm 60 such that the surgical instrument 4 and the manipulator arm 60 do not enter the contact prohibited space VPC. The controller 31 may cause the manipulator arm 60 and the arm base 50 to operate so as to perform the contact prohibition control such that the surgical instrument 4 and the manipulator arm 60 do not enter the contact prohibited space VPC.

The controller 31 calculates the postures of the surgical instrument 4 and the manipulator arm 60 based on a surgeon's operation command value received by the operation manipulator arm 21, the rotation angles extracted from the encoders E4 of the positioner 40, and the rotation angles extracted from the encoders E1 and E3 of the manipulator arm 60 holding the endoscope 6. When in the calculated postures, the surgical instrument 4 and the manipulator arm 60 enter the contact prohibited space VPC, the controller 31 performs a control to notify the surgeon that the surgical instrument 4 and the manipulator arm 60 are in the contact prohibited space VPC, not receive the operation, and maintain the current values of the rotation angles detected by the encoders E1 (E2, E3), or performs a control to change a path of the manipulator arm 60 such that the surgical instrument 4 and the manipulator arm 60 do not enter the contact prohibited space VPC to operate the surgical instrument 4. The controller 31 also calculates the postures of the surgical instrument 4 and the manipulator arm 60 based on an operator's operation command value received by the operation unit 80 and the rotation angles extracted from the encoders E4 of the positioner 40. When in the calculated postures, the surgical instrument 4 and the manipulator arm 60 enter the contact prohibited space VPC, the controller 31 notifies the operator that the surgical instrument 4 and the manipulator arm 60 are in the contact prohibited space VPC, but performs a control to operate the surgical instrument 4 and the manipulator arm 60 based on the operation command value received by the operation unit 80. That is, when the manipulator arm 60 is operated through the operation manipulator arm 21, the controller 31 performs the contact prohibition control to operate the manipulator arm 60 such that the surgical instrument 4 and the manipulator arm 60 do not enter the contact prohibited space VPC. On the other hand, when the manipulator arm 60 is operated through the operation unit 80, the controller 31 performs a control to operate the manipulator arm 60 based on an operation on the operation unit 80 even when the surgical instrument 4 and the manipulator arm 60 enter the contact prohibited space VPC.

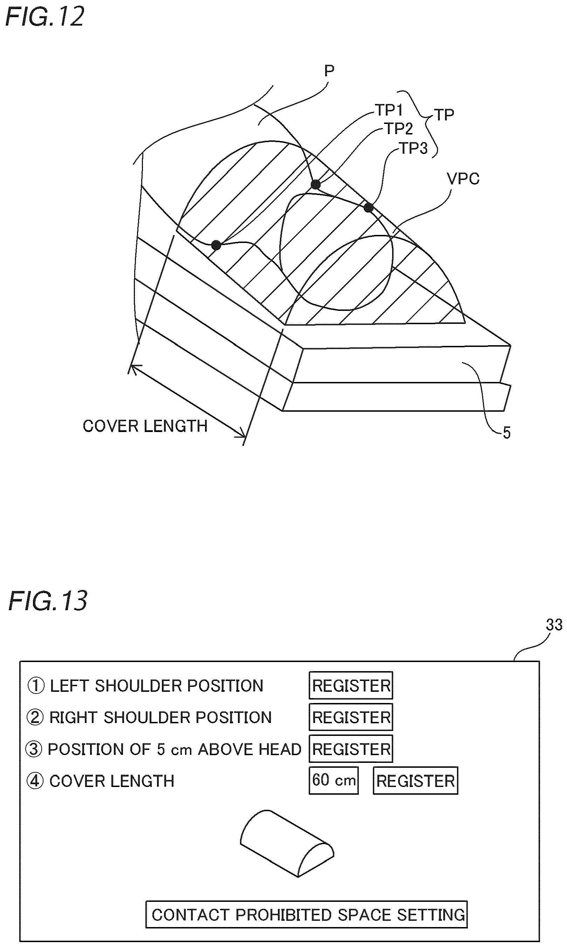

As shown in FIG. 12, the endoscope 6 (see FIG. 6) or the pivot teaching member 7 (see FIG. 7) as a teaching unit and the operation unit 80 (see FIG. 5) teach teaching points TP (TP1, TP2, and TP3) in a space in which the manipulator arm 60 operates. Specifically, the contact prohibited space VPC is set based on the teaching points TP taught by the pivot teaching member 7 (see FIG. 7) attached to the tip end side of the manipulator arm 60 operated through the operation unit 80, or the tip end 6a (see FIG. 6) of the endoscope 6 attached to the tip end side of the manipulator arm 60. That is, the operation unit 80 is operated such that the tip end 7a of the pivot teaching member 7 attached to the tip end side of the manipulator arm 60 or the tip end 6a of the endoscope 6 attached to the tip end side of the manipulator arm 60 is moved to a position to be taught, and the teaching point TP is taught.

In this embodiment, the controller 31 sets the virtual contact prohibited space VPC in the space around the contact prohibited object (patient P) based on the teaching points TP taught using the teaching unit (the endoscope 6 or the pivot teaching member 7, the operation unit 80). In this embodiment, the controller 31 performs the contact prohibition control to operate the manipulator arm 60 such that the surgical instrument 4 and the manipulator arm 60 do not enter the set contact prohibited space VPC.

The controller 31 sets the contact prohibited space VPC based on the teaching points TP taught using the manipulator arm 60 operated through the operation unit 80. Specifically, the controller 31 sets the contact prohibited space VPC based on the teaching points TP taught by the pivot teaching member 7 attached to the tip end side of the manipulator arm 60 operated through the operation unit 80 or the tip end 6a of the endoscope 6 attached to the tip end side of the manipulator arm 60.

As shown in FIG. 12, the controller 31 sets the contact prohibited space VPC to cover the periphery of the patient P as the contact prohibited object. Specifically, the controller 31 sets the contact prohibited space VPC to cover the periphery of a site on the patient P other than the surgical site. The controller 31 also sets the contact prohibited space VPC to cover the periphery of a site on the patient P on the side on which the manipulator arm 60 is arranged (deployed) according to the surgical site. For example, when the abdomen is the surgical site, the controller 31 sets the contact prohibited space VPC to cover the periphery of the head of the patient P. When the head is the surgical site, the controller 31 sets the contact prohibited space VPC to cover the periphery of the upper body of the patient P.

As shown in FIG. 12, the controller 31 sets the contact prohibited space VPC that covers the periphery of the head of the patient P based on the teaching points TP including the left shoulder of the patient P, the right shoulder of the patient P, and a position at a predetermined distance from the head. Specifically, the controller 31 sets the contact prohibited space VPC based on the teaching point TP1 at the left shoulder position, the teaching point TP2 at the right shoulder position, and the teaching point TP3 at 5 cm above the head.

The controller 31 also sets the contact prohibited space VPC based on the teaching points TP (TP1, TP2, and TP3) and an input operation for the length of the patient P in the body axis direction (cover length). When the contact prohibited space VPC is set, an operator's registration operation is received by the input device 33 and the touch panel 23. Specifically, an operation to register the teaching points TP taught by the teaching unit (the endoscope 6 or the pivot teaching member 7, the operation unit 80) is received by the input device 33 and the touch panel 23. The input operation for the length of the patient P in the body axis direction (cover length) is received by the input device 33 and the touch panel 23. It may be possible to switch which of the input device 33 and the touch panel 23 receives an operation.

As shown in FIG. 12, the controller 31 sets a semi-cylindrical contact prohibited space VPC around the contact prohibited object (patient P). Specifically, the controller 31 determines the diameter of the semi-cylindrical shape and the position of the semi-cylindrical shape based on both shoulder positions and a position of 5 cm above the head as viewed in the body axis direction of the patient P, determines the axial length of the semi-cylindrical shape from the shoulder position based on the cover length, and sets the contact prohibited space VPC.

The controller 31 performs a control to issue a warning when at least one of the surgical instrument 4 and the manipulator arm 60 enters the contact prohibited space VPC. Specifically, the controller 31 performs a control to display a warning on the monitor 24 of the remote control apparatus 2 and the display 8a of the monitor cart 8 when at least one of the surgical instrument 4 and the manipulator arm 60 enters the contact prohibited space VPC. The controller 31 performs a control to emit an alarm sound that warns that at least one of the surgical instrument 4 and the manipulator arm 60 has entered the contact prohibited space VPC from speakers (not shown) built into the remote control apparatus 2, the medical cart 3, and the monitor cart 8 when at least one of the surgical instrument 4 and the manipulator arm 60 has entered the contact prohibited space VPC.

The controller 31 performs the contact prohibition control to operate the plurality of manipulator arms 60 such that a plurality of surgical instruments 4 and the plurality of manipulator arms 60 do not enter the contact prohibited space VPC. Specifically, the controller 31 sets the contact prohibited space VPC based on the teaching points TP taught using one of the plurality of manipulator arms 60 operated through one of a plurality of operation units 80 provided on the plurality of manipulator arms 60, respectively. Then, the controller 31 performs the contact prohibition control to operate the plurality of manipulator arms 60 such that the plurality of surgical instruments 4 and the plurality of manipulator arms 60 do not enter the contact prohibited space VPC.

### Contact Prohibited Space Setting Procedure

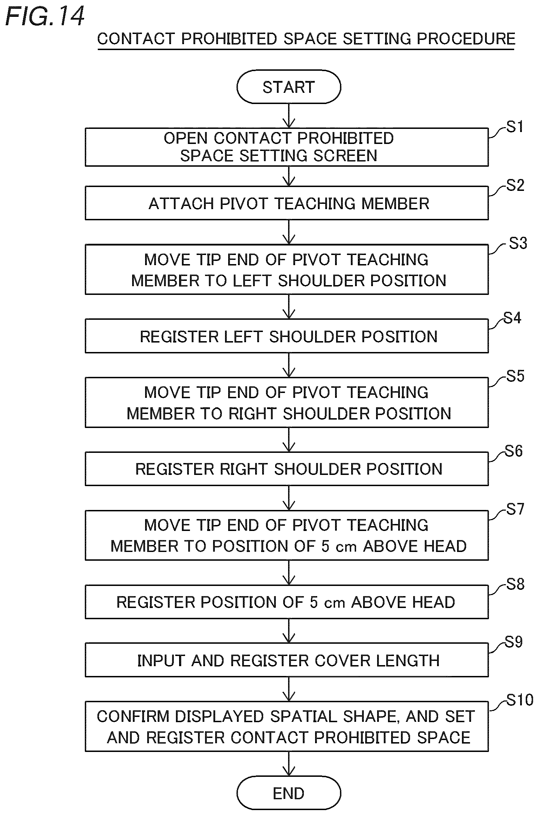

A procedure for setting the contact prohibited space VPC is now described with reference to FIGS. 13 and 14. The contact prohibited space VPC is set before the pivot position PP is set. That is, this is because when the pivot position PP is set, the manipulator arm 60 is constrained at the pivot position PP and cannot move freely.

In step S1 of FIG. 14, the operator performs an operation to open a contact prohibited space setting screen (see FIG. 13). The operation is received by the input device 33 of the medical cart 3 or the touch panel 23 of the remote control apparatus 2. In step S2, the operator attaches the pivot teaching member 7 to the tip end of the manipulator arm 60. When the tip end of the endoscope 6 is used to teach the teaching points TP, the pivot teaching member 7 does not have to be attached at this point.

In step S3, the operator operates the operation unit 80 to move the tip end 7a (see FIG. 7) of the pivot teaching member 7 to the position of the teaching point TP1 (see FIG. 12) at the left shoulder of the patient P. In step S4, the operator performs an operation to register the left shoulder position on an operation screen (see FIG. 13).

In step S5, the operator operates the operation unit 80 to move the tip end 7a (see FIG. 7) of the pivot teaching member 7 to the position of the teaching point TP2 (see FIG. 12) at the right shoulder of the patient P. In step S6, the operator performs an operation to register the right shoulder position on the operation screen (see FIG. 13).

In step S7, the operator operates the operation unit 80 to move the tip end 7a (see FIG. 7) of the pivot teaching member 7 to the position of the teaching point TP3 (see FIG. 12) at 5 cm above the head of the patient P. In step S8, the operator performs an operation to register the position of 5 cm above the head on the operation screen (see FIG. 13).

In step S9, the operator inputs the cover length to perform an operation to register the cover length. Then, in step S10, the spatial shape of the displayed contact prohibited space VPC is confirmed, and an operation to set and resister the contact prohibited space VPC is performed.

### Contact Prohibited Space Confirmation Procedure

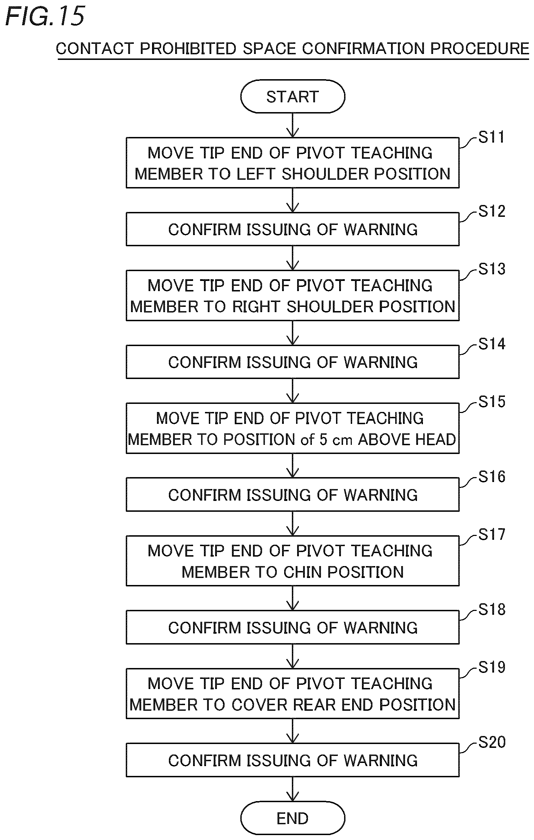

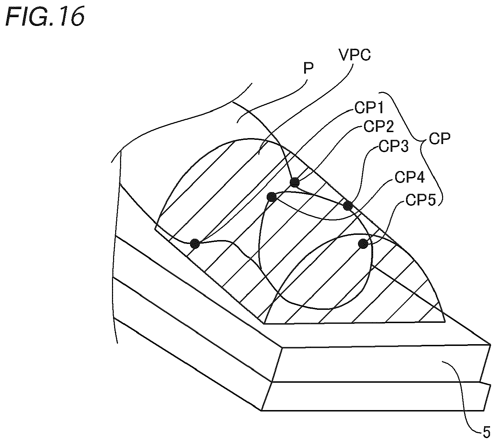

A procedure for confirming the contact prohibited space VPC is now described with reference to FIGS. 15 and 16.

In step S11 of FIG. 15, the operator operates the operation unit 80 to move the tip end 7a (see FIG. 7) of the pivot teaching member 7 to the left shoulder position CP1 (see FIG. 16) of the patient P. The left shoulder position CP1 is within the contact prohibited space VPC, and thus the controller 31 performs a control to display a warning that the pivot teaching member 7 has entered the contact prohibited space VPC on the monitor 24 of the remote control apparatus 2 and the display 8a of the monitor cart 8. Furthermore, the controller 31 performs a control to emit an alarm sound to warn that the pivot teaching member 7 has entered the contact prohibited space VPC. In step S12, the operator confirms that the warning has been normally issued.

In step S13, the operator operates the operation unit 80 to move the tip end 7a (see FIG. 7) of the pivot teaching member 7 to the right shoulder position CP2 (see FIG. 16) of the patient P. The right shoulder position CP2 is within the contact prohibited space VPC, and thus the controller 31 performs a control to display a warning that the pivot teaching member 7 has entered the contact prohibited space VPC on the monitor 24 of the remote control apparatus 2 and the display 8a of the monitor cart 8. Furthermore, the controller 31 performs a control to emit an alarm sound to warn that the pivot teaching member 7 has entered the contact prohibited space VPC. In step S14, the operator confirms that the warning has been normally issued.

In step S15, the operator operates the operation unit 80 to move the tip end 7a (see FIG. 7) of the pivot teaching member 7 to the position CP3 (see FIG. 16) of 5 cm above the head of the patient P. The position CP3 of 5 cm above the head of the patient P is within the contact prohibited space VPC, and thus the controller 31 performs a control to display a warning that the pivot teaching member 7 has entered the contact prohibited space VPC on the monitor 24 of the remote control apparatus 2 and the display 8a of the monitor cart 8. Furthermore, the controller 31 performs a control to emit an alarm sound to warn that the pivot teaching member 7 has entered the contact prohibited space VPC. In step S16, the operator confirms that the warning has been normally issued.

In step S17, the operator operates the operation unit 80 to move the tip end 7a (see FIG. 7) of the pivot teaching member 7 to the chin position CP4 (see FIG. 16) of the patient P. The chin position CP4 is within the contact prohibited space VPC, and thus the controller 31 performs a control to display a warning that the pivot teaching member 7 has entered the contact prohibited space VPC on the monitor 24 of the remote control apparatus 2 and the display 8a of the monitor cart 8. Furthermore, the controller 31 performs a control to emit an alarm sound to warn that the pivot teaching member 7 has entered the contact prohibited space VPC. In step S18, the operator confirms that the warning has been normally issued.

In step S19, the operator operates the operation unit 80 to move the tip end 7a (see FIG. 7) of the pivot teaching member 7 to a cover rear end position CP5 (see FIG. 16) (a position on the head direction side) within the reach of the tip end 7a of the pivot teaching member 7. The cover rear end position CP5 is within the contact prohibited space VPC, and thus the controller 31 performs a control to display a warning that the pivot teaching member 7 has entered the contact prohibited space VPC on the monitor 24 of the remote control apparatus 2 and the display 8a of the monitor cart 8. Furthermore, the controller 31 performs a control to emit an alarm sound to warn that the pivot teaching member 7 has entered the contact prohibited space VPC. In step S20, the operator confirms that the warning has been normally issued. After that, the procedure for confirming the contact prohibited space VPC is terminated. After that, the pivot position is taught by the pivot teaching member 7.

The functionality of each controller disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out or is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Advantages of This Embodiment

According to this embodiment, the following advantages are achieved.

According to this embodiment, as described above, the controller 31 is configured or programmed to set the virtual contact prohibited space VPC in the space around the contact prohibited object (patient P) based on the teaching points TP taught using the teaching unit (the endoscope 6 or the pivot teaching member 7, the operation unit 80). Accordingly, unlike a case in which a plane passing through the pivot position PP is set as a surface of an obstacle based on the pivot position PP that serves as a fulcrum for movement of the surgical instrument 4, the contact prohibited space VPC can be set at an arbitrary position with respect to the pivot position PP. Thus, the degree of freedom in setting a contact prohibited range for prohibiting the surgical instrument 4 and the manipulator arm 60 from contacting the contact prohibited object (patient P) can be improved. Furthermore, the virtual contact prohibited space VPC is set in the space around the contact prohibited object (patient P) based on the teaching points TP taught using the teaching unit (the endoscope 6 or the pivot teaching member 7, the operation unit 80) such that contact prohibited spaces VPC having suitable shapes and sizes can be easily set for contact prohibited objects having various shapes and sizes. Consequently, the degree of freedom in setting the contact prohibited range for prohibiting the surgical instrument 4 and the manipulator arm 60 from contacting the contact prohibited object can be improved, and the contact prohibited range can be easily set.

According to this embodiment, as described above, the controller 31 is configured or programmed to set the contact prohibited space VPC based on the teaching points TP taught using the manipulator arm 60 operated through the operation unit 80. Accordingly, the teaching points TP can be taught while the manipulator arm 60 is moved by operating the manipulator arm 60 through the operation unit 80, and thus the positions of the teaching points TP can be taught easily and accurately.

According to this embodiment, as described above, the controller 31 is configured or programmed to set the contact prohibited space VPC based on the teaching points TP taught by the pivot teaching member 7 attached to the tip end side of the manipulator arm 60 operated through the operation unit 80 or the tip end 6a of the endoscope 6 attached to the tip end side of the manipulator arm 60. Accordingly, it is not necessary to separately provide a dedicated member for teaching the teaching points TP for setting the contact prohibited space VPD, and thus an increase in the number of components can be reduced or prevented, and the complexity of the apparatus configuration can be reduced or prevented.

According to this embodiment, as described above, the controller 31 is configured or programmed to set the contact prohibited space VPC to cover the periphery of the patient P as the contact prohibited object. Accordingly, unlike a case in which the patient P is covered with a cradle (cover) to prevent the surgical instrument 4 and the manipulator arm 60 from contacting the patient P, the surgical instrument 4 and the manipulator arm 60 can be prevented from contacting the patient P by setting the virtual contact prohibited space VPC around the patient P. Thus, the surgical instrument 4 and the manipulator arm 60 do not come the cradle. Consequently, damage and misalignment of the surgical instrument 4 and the manipulator arm 60 due to contact of the surgical instrument 4 and the manipulator arm 60 with the cradle can be reduced or prevented.

According to this embodiment, as described above, the controller 31 is configured or programmed to set the contact prohibited space VPC to cover the periphery of the site on the patient P other than the surgical site. Accordingly, the surgical instrument 4 can be inserted into the surgical site on the patient P, and contact of the surgical instrument 4 and the manipulator arm 60 with the site on the patient P other than the surgical site can be reduced or prevented.

According to this embodiment, as described above, the controller 31 is configured or programmed to set the contact prohibited space VPC to cover the periphery of the head of the patient P. Accordingly, when surgery is performed by inserting the surgical instrument 4 into the abdomen of the patient P, contact of the manipulator arm 60 with the head of the patient P can be reduced or prevented when the surgical instrument 4 is tilted such that the manipulator arm 60 is positioned on the head side in order to exfoliate the peritoneum of the patient P, for example.

According to this embodiment, as described above, the controller 31 is configured or programmed to set the contact prohibited space VPC that covers the periphery of the head of the patient P based on the teaching points TP including the left shoulder of the patient P, the right shoulder of the patient P, and the position at the predetermined distance from the head of the patient P. Accordingly, the contact prohibited space VPC can be set according to the lateral length of the head of the patient P based on the teaching points of the shoulders of the patient P. Furthermore, the contact prohibited space VPC can be set according to the height of the head of the patient P based on the teaching point TP at the predetermined distance from the head of the patient P.

According to this embodiment, as described above, the controller 31 is configured or programmed to set the contact prohibited space VPC based on the teaching points TP taught using the teaching unit (the endoscope 6 or the pivot teaching member 7, the operation unit 80) and the input operation for the length of the patient P in the body axis direction. Accordingly, the contact prohibited space VPC can be set according to the length of the head of each patient P in the body axis direction by inputting the length of the patient P in the body axis direction.

According to this embodiment, as described above, the controller 31 is configured or programmed to set the semi-cylindrical contact prohibited space VPC around the contact prohibited object (patient P). Accordingly, the contact prohibited space VPC can be formed into a relatively simple semi-cylindrical shape, and thus the complexity of the contact prohibited space VPC setting process can be reduced or prevented.

According to this embodiment, as described above, the controller 31 is configured or programmed to perform a control to issue a warning when at least one of the surgical instrument 4 and the manipulator arm 60 enters the contact prohibited space VPC. Accordingly, the operator can easily confirm whether or not at least one of the surgical instrument 4 and the manipulator arm 60 has entered the contact prohibited space VPC.

According to this embodiment, as described above, the operation unit 80 includes the joystick 82 to manipulate the moving direction of the manipulator arm 60. Accordingly, the manipulator arm 60 can be easily moved to a desired position by operating the joystick 82, and thus the manipulator arm 60 can be used to easily teach the teaching points TP for setting the contact prohibited space VPC.

According to this embodiment, as described above, the operation unit 80 includes the enable switches 81 to enable movement of the manipulator arm 60 when the enable switches 81 are pressed. Accordingly, movement of the manipulator arm 60 through the operation unit 80 is enabled by pressing the enable switches 81, and thus unintended movement of the manipulator arm 60 when the enable switches 81 are not pressed can be reduced or prevented.

According to this embodiment, as described above, the input device 33 and the touch panel 23 are provided to register the teaching points TP taught by the teaching unit (the endoscope 6 or the pivot teaching member 7, the operation unit 80). Accordingly, the teaching points TP taught by the teaching unit (the endoscope 6 or the pivot teaching member 7, the operation unit 80) can be easily registered by operating the input device 33 and the touch panel 23.

According to this embodiment, as described above, the manipulator arm 60 includes the seven or more joint axes. The posture of the manipulator arm 60 for maintaining the pivot position PP that serves as a fulcrum for movement of the surgical instrument 4 can be determined by the amount of rotation (movement) of the six joint axes of the manipulator arm 60. Therefore, the manipulator arm 60 includes the seven or more joint axes such that a redundant axis is generated. That is, the manipulator arm 60b can take a different posture while the pivot position PP is maintained. Therefore, the manipulator arm 60 includes the seven or more joint axes such that the posture of the manipulator arm 60b can be changed to reduce or prevent interference with the manipulator arm 60c while the pivot position PP is maintained. Furthermore, the manipulator arm 60 includes the seven or more joint axes such that the posture of the manipulator arm 60 can be changed to avoid the contact prohibited space VPC while the pivot position PP is maintained.

According to this embodiment, as described above, the controller 31 is configured or programmed to perform the contact prohibition control to operate the manipulator arm 60 such that the surgical instrument 4 and the manipulator arm 60 do not enter the contact prohibited space VPC when the manipulator arm 60 is operated through the operation manipulator arm 21. Furthermore, the controller 31 is configured or programmed to perform a control to operate the manipulator arm 60 based on the operation on the operation unit 80 when the manipulator arm 60 is operated through the operation unit 80. Accordingly, even when the surgical instrument 4 or the manipulator arm 60 unintentionally enters the contact prohibited space VPC for some reason, the manipulator arm 60 can be operated through the operation unit 80, and thus the surgical instrument 4 or the manipulator arm 60 can be taken out of the contact prohibited space VPC.

### Modified Examples

The embodiment disclosed this time must be considered as illustrative in all points and not restrictive. The scope of the present disclosure is not shown by the above description of the embodiment but by the scope of claims for patent, and all modifications (modified examples) within the meaning and scope equivalent to the scope of claims for patent are further included.

For example, while the example in which the manipulator arm is moved through the operation unit to teach the teaching points for setting the contact prohibited space has been shown in the aforementioned embodiment, the present disclosure is not limited to this. A laser beam generator and a camera may be provided in the manipulator arm, the arm base, or the positioner, the teaching points may be taught using laser beams generated from the laser beam generator, and the controller may acquire the teaching points based on imaging of the laser beam by the camera. Alternatively, a camera may be provided in the manipulator arm, the arm base, or the positioner, the contact prohibited object may be recognized based on an image captured by the camera, and the contact prohibited space may be set around the recognized contact prohibited object.

While the example in which the semi-cylindrical contact prohibited space is set has been shown in the aforementioned embodiment, the present disclosure is not limited to this. For example, the contact prohibited space may have a polygonal prismatic shape, a conical shape, or a polygonal conical shape.

While the example in which the contact prohibited object is a patient has been shown in the aforementioned embodiment, the present disclosure is not limited to this. For example, the contact prohibited object may be an operating table, equipment in the vicinity of the operating table, or equipment attached to a patient.

While the example in which the contact prohibited space that covers the periphery of the head of the patient is set based on the teaching points including the left shoulder of the patient, the right shoulder of the patient, and the position at the predetermined distance from the head has been shown in the aforementioned embodiment, the present disclosure is not limited to this. The contact prohibited space that covers the periphery of the head of the patient may be set based on the teaching point including at least one of the left shoulder of the patient, the right shoulder of the patient, and the position at the predetermined distance from the head. Alternatively, the contact prohibited space may be set using the position of another site on the patient, such as the ears, the nose, the jaw, or the top of the head of the patient, as a teaching point.

While the example in which the teaching points for setting the contact prohibited space are registered based on the operations on the input device and the touch panel provided separately from the operation unit that moves the manipulator arm has been shown in each of the aforementioned embodiment, the present disclosure is not limited to this. A button for registering the teaching points may be provided on the operation unit that moves the manipulator arm, and the teaching points for setting the contact prohibited space may be registered based on an operation on the operation unit.

While the example in which the pivot teaching member or the endoscope is used to teach the teaching points for setting the contact prohibited space has been shown in the aforementioned embodiment, the present disclosure is not limited to this. The teaching points for setting the contact prohibited space may be taught using a surgical instrument such as a pair of surgical forceps attached to the tip end of the manipulator arm.

While the example in which four manipulator arms are provided has been shown in the aforementioned embodiment, the present disclosure is not limited to this. The number of manipulator arms may be any number as long as at least one manipulator arm is provided.

While the example in which each of the arm portion and the positioner includes a 7-axis articulated robot has been shown in the aforementioned embodiment, the present disclosure is not limited to this. For example, each of the arm portion and the positioner may include an articulated robot having an axis configuration (six axes or eight axes, for example) other than the 7-axis articulated robot.

While the example in which all of the manipulator arms include a 7-axis articulated robot has been shown in the aforementioned embodiment, the present disclosure is not limited to this. For example, some of the four manipulator arms may include a 7-axis articulated robot.

While the example in which the medical manipulator includes the medical cart, the positioner, the arm base, and the manipulator arms has been shown in the aforementioned embodiment, the present disclosure is not limited to this. For example, the medical manipulator may not include the medical cart, the positioner, or the arm base, but may include only the manipulator arms.

### Description of Reference Numerals

1: medical manipulator (surgical robot, patient-side apparatus)
2: remote control apparatus (operator-side apparatus)
4: surgical instrument
6: endoscope (teaching member, surgical instrument)
7: pivot teaching member (teaching unit)
23: touch panel (input)
31: controller
33: input device (input)
60, 60a, 60b, 60c, 60d: manipulator arm
80: operation unit (teaching unit)
81: enable switch
82: joystick
100: robotic surgical system
PP: pivot position
TP, TP1, TP2, TP3: teaching point

## Claims

1. A surgical robot comprising:
a manipulator arm having a tip end side to which a surgical instrument is attached;
a controller configured or programmed to perform a control to operate the manipulator arm to which the surgical instrument is attached; and
a teaching unit to teach a teaching point in a space in which the manipulator arm operates; wherein
the controller is configured or programmed to set a virtual contact prohibited space in a space around a contact prohibited object based on a plurality of the teaching points taught using the teaching unit.

2. The surgical robot according to claim 1, wherein the teaching unit includes an operation unit provided on the manipulator arm to operate the manipulator arm.

3. The surgical robot according to claim 2, wherein the controller is configured or programmed to set the contact prohibited space based on the teaching points taught using the manipulator arm operated through the operation unit.

4. The surgical robot according to any one of claims 1 to 3, wherein
the teaching unit further includes at least one of a pivot teaching member attached to the tip end side of the manipulator arm to teach a pivot position that serves as a fulcrum for movement of the surgical instrument, and an endoscope attached to the tip end side of the manipulator arm to image a surgical site; and
the controller is configured or programmed to set the contact prohibited space based on the teaching points taught by the pivot teaching member attached to the tip end side of the manipulator arm or a tip end of the endoscope attached to the tip end side of the manipulator arm.

5. The surgical robot according to any one of claims 1 to 4, wherein the controller is configured or programmed to set the contact prohibited space to cover a periphery of a patient as the contact prohibited object.

6. The surgical robot according to claim 5, wherein the controller is configured or programmed to set the contact prohibited space to cover a periphery of a site on the patient other than a surgical site.

7. The surgical robot according to claim 5 or 6, wherein the controller is configured or programmed to set the contact prohibited space based on the teaching points taught using the teaching unit and an input operation for a length of the patient in a body axis direction.

8. The surgical robot according to any one of claims 1 to 7, wherein the controller is configured or programmed to set the contact prohibited space having a semi-cylindrical shape around the contact prohibited object.

9. The surgical robot according to any one of claims 1 to 8, wherein the controller is configured or programmed to perform a control to issue a warning when at least one of the surgical instrument and the manipulator arm enters the contact prohibited space.

10. The surgical robot according to any one of claims 1 to 8, wherein the controller is configured or programmed to perform a contact prohibition control to operate the manipulator arm such that the surgical instrument and the manipulator arm do not enter the contact prohibited space.

11. The surgical robot according to claim 2 and any one of claims 3 to 10 as dependent on claim 2, wherein the operation unit includes a joystick to manipulate a moving direction of the manipulator arm.

12. The surgical robot according to claim 2 and any one of claims 3 to 11 as dependent on claim 2, wherein the operation unit includes an enable switch to enable movement of the manipulator arm when the enable switch is pressed.

13. The surgical robot according to any one of claims 1 to 12, further comprising:
an input to register the teaching point taught by the teaching unit.

14. The surgical robot according to any one of claims 1 to 13, wherein the manipulator arm includes seven or more joint axes.

15. The surgical robot according to any one of claims 1 to 9, wherein
the manipulator arm includes a plurality of manipulator arms having tip end sides to which surgical instruments are attached, respectively; and
the controller is configured or programmed to perform a contact prohibition control to operate the plurality of manipulator arms such that the surgical instruments and the plurality of manipulator arms do not enter the contact prohibited space.

16. The surgical robot according to claim 3 and any one of claims 4 to 9 as dependent on claim 3, wherein
the manipulator arm includes a plurality of manipulator arms having tip end sides to which surgical instruments are attached, respectively;
the operation unit includes a plurality of operation units provided on the plurality of manipulator arms, respectively;
the controller is configured or programmed to set the contact prohibited space based on the teaching points taught using one of the plurality of manipulator arms operated through one of the plurality of operation units; and
the controller is configured or programmed to perform a contact prohibition control to operate the plurality of manipulator arms such that the surgical instruments and the plurality of manipulator arms do not enter the contact prohibited space.

17. A robotic surgical system comprising:
a patient-side apparatus including a manipulator arm having a tip end side to which a surgical instrument is attached, and a teaching unit to teach a teaching point in a space in which the manipulator arm operates;
an operator-side apparatus to receive an operation for the surgical instrument; and
a controller configured or programmed to perform a control to operate the manipulator arm to which the surgical instrument is attached; wherein
the controller is configured or programmed to set a virtual contact prohibited space in a space around a contact prohibited object based on a plurality of the teaching points taught using the teaching unit.

18. The robotic surgical system according to claim 17, wherein
the teaching unit includes an operation unit provided on the manipulator arm to operate the manipulator arm; and
the controller is configured or programmed to:
perform a contact prohibition control to operate the manipulator arm such that the surgical instrument and the manipulator arm do not enter the contact prohibited space when the manipulator arm is operated through the operator-side apparatus; and
perform a control to operate the manipulator arm based on an operation on the operation unit even when the surgical instrument and the manipulator arm enter the contact prohibited space when the manipulator arm is operated through the operation unit.

19. A control method for a surgical robot, the control method comprising:
setting a virtual contact prohibited space in a space around a contact prohibited object based on a plurality of teaching points taught using a teaching unit to teach the teaching points in a space in which a manipulator arm having a tip end side to which a surgical instrument is attached operates; and
controlling operation of the manipulator arm such that the surgical instrument and the manipulator arm do not enter the set contact prohibited space.
